# EUROPÄISCHE PATENTSCHRIFT

(11) **EP 1 931 282 B1**
(45) Veröffentlichungstag und Bekanntmachung des Hinweises auf die Patenterteilung: **12.12.2012**
(21) Anmeldenummer: 06806002.9
(22) Anmeldetag: 02.10.2006
(51) Int. Cl.: A61F 2/06, A61F 2/88, A61F 2/84

(54) **STENT ZUM EINFÜHREN IN MENSCHLICHE KÖRPERHÖHLEN INSBESONDERE IN BLUTGEFÄSSE**
STENT FOR INTRODUCING INTO HUMAN BODY CAVITIES, IN PARTICULAR INTO BLOOD VESSELS
ENDOPROTHESE A INTRODUIRE DANS DES CAVITES DU CORPS HUMAIN, NOTAMMENT DANS DES VAISSEAUX SANGUINS

(30) Priorität: 30.09.2005 DE 102005047431; 29.10.2005 DE 102005052226
(43) Veröffentlichungstag der Anmeldung: 18.06.2008
(73) Patentinhaber: Friebe, Michael, 45657 Recklinghausen (DE); Baumgart, Dietrich, 45133 Essen (DE)
(72) Erfinder: Friebe, Michael, 45657 Recklinghausen (DE); Baumgart, Dietrich, 45133 Essen (DE)
(74) Vertreter: Gesthuysen, von Rohr & Eggert
(86) Internationale Anmeldenummer: PCT/EP2006/009560
(87) Internationale Veröffentlichungsnummer: WO 2007/039273

(56) Entgegenhaltungen:
- EP-A2- 0 947 180
- WO-A-01/41676
- WO-A-90/04982
- DE-A1- 19 703 482
- DE-U1-202005 001 416
- US-A- 4 512 338

## Beschreibung

Die Erfindung betrifft einen Stent mit den Merkmalen des Oberbegriffs von Anspruch 1.

Aus der US 4,512,338 A ist ein Stent bekannt, der einen als formhaltenden Federdraht ausgebildeten Stützdraht aufweist, wobei der Stützdraht im komprimierten Zustand im Bereich des Stentkörpers vorgespannt und in eine Linienform gezogen ist und der sich beim Einfedern zumindest abschnittsweise in eine Spiralform in Längsrichtung zusammenzieht und radial spiralförmig expandiert. Aus der EP 0 947 180 A2 ist ein weiterer Stent mit einem spiralförmigen Stützdraht bekannt.

Aus dem Stand der Technik sind Stents der zuvor beschriebenen Art, die zum Teil auch als Prothesen bezeichnet werden, bekannt. Solche Stents dienen üblicherweise dazu, den Strömungsquerschnitt des Lumens eines (Blut-)Gefäßes zu vergrößern oder dauerhaft offen zu halten. Darüber hinaus werden Stents im Zusammenhang mit der endovaslukären Behandlung abdominaler Aortenaneurysmen (AAA) zur Abdichtung von Aneurysmen (Aussackungen) eingesetzt.

Bekannte Stents sind dabei üblicherweise so ausgebildet, daß sie der Neigung des Gefäßes, sich zu verengen, widerstehen, so daß die Durchgängigkeit des Gefäßes gewahrt bleibt. Sofern der Stent zur Abdichtung eines Aneurysmas vorgesehen ist, muß darüber hinaus sichergestellt sein, daß es nicht zu einem Einstülpen des Stents in die Aussackung kommt. Typische expandierbare Stents weisen im allgemeinen eine röhren- bzw. hülsenartige Form auf, und sind von einem zusammengefalteten, komprimierten Zustand auf einen Aufspannzustand expandierbar. Zur Implantation wird ein komprimierter Stent üblicherweise mit Hilfe eines Katheters an die gewünschte Implantationsstelle gebracht. Dort wird der Stent aus dem Katheter geschoben oder von dem Katheter abgeschoben und expandiert bzw. aufgefaltet. Dies kann durch Selbst-expandieren oder über Hilfsmittel erfolgen. Bei sogenannten selbstexpandierenden Stents wird der Stent über einen Einführkatheter an die Implantationsstelle gebracht. Anschließend wird der über den Stent geschobene Einführkatheter oder eine äußere Schutzhülle bei einem auf den Einflihrkatheter aufgeschobenen Stent abgezogen, was zu einer Expansion des Stents durch Einfedern des Stützkörpers in die Aufspannstellung führt, Bei nicht selbstexpandierenden Stents wird die Expansion üblicherweise über einen im Inneren des Stents vorgesehenen Ballon vorgenommen, in den in der Regel eine Lösung eingebracht wird, so daß der Ballon sich aufbläht und damit den Stent aufspannt. Nach einer anschließenden Entleerung des Ballons kann dieser aus dem Stent herausgezogen werden.

Aus der DE 20 2005 001 416 ist bereits ein Stent der eingangs beschriebenen Art bekannt, der als Stützgerüst mindestens eine umlaufende unterbrochene und/oder nicht-unterbrochene Spiralfeder aufweist. Die Spiralfeder verstärkt die Dehnbarkeit des Stents in Längsrichtung, was zu einem leichteren Ein- und Ausführen des Stents in und aus einer Körperhöhle beiträgt. Der Stentkörper und das metallische Stützgerüst sind dabei in der Richtung senkrecht zur Längsachse des Stents nicht-expandierend ausgebildet. Von Nachteil ist, daß sich der bekannte Stent zum Teil nur mit erheblichem Aufwand oder gar nicht in eine Körperhöhle mit einem geringen Lumen einführen läßt. Darüber hinaus kommt es bei dem bekannten Stent im Aufspannzustand leicht zu einer ungewollten Verschiebung in der Körperhöhle, was ebenfalls von Nachteil ist.

Aufgabe der vorliegenden Erfindung ist es, einen gattungsgemäßen Stent bereitzustellen, welcher sich einfach in eine Körperhöhle einführen läßt und eine Stützfunktion des umgebenden Gewebes der Körperhöhle erfüllt, ohne daß es zu einer ungewollten Verschiebung des Stents in der Körperhöhle im Aufspannzustand kommen kann.

Zur Lösung der vorgenannten Aufgabe wird bei einer ersten alternativen Ausführungsform der Erfindung ein Stent mit den Merkmalen von Anspruch 1 vorgeschlagen. Zunächst darf darauf hingewiesen werden, daß der Ausdruck "proximal" die Bedeutung von "zum Herzen hin" hat, während der Ausdruck "distal" die Bedeutung von "vom Herzen weg" hat. Die Formstabilität des Stents im Aufspannzustand wird durch das Material des Stentkörpers und das Material des Stützdrahtes sowie dessen Federkraft vorgegeben. Dadurch wird sichergestellt, daß der Stent der Neigung des Gefäßes, sich zu verengen, widersteht und daß im Fall der Behandlung eines abdominalen Aortenaneurysmas nicht zu einem Einstülpen des Stentkörpers in die Aussackung kommen kann.

Der erfindungsgemäße Stent bietet eine Reihe von wesentlichen Vorteilen. Wichtig ist in diesem Zusammenhang zunächst, daß der Stützdraht im komprimierten Zustand des Stents, der auch als Einführzustand bezeichnet werden kann, in Längsrichtung gestreckt bzw. vorgespannt ist, so daß der Stützdraht im komprimierten Zustand vorzugsweise im wesentlichen langgestreckt bis gerade, gegebenenfalls leicht gewunden mit großem Windungsabstand, ausgebildet ist. Im Ergebnis wird zum Einführen des erfindungsgemäßen Stents in eine Körperhöhle der Stützdraht im wesentlichen vollständig in eine gerade Linienform gezogen bzw. vorgespannt. Dadurch ist es möglich, beim Einführen des Stents in eine Körperhöhle den Stentkörper auf einen sehr kleinen Durchmesser zusammenzulegen, wobei der Stentkörper sehr enganliegend um den langgestreckten Stützdraht gewickelt werden kann, was das Einführen deutlich vereinfacht. Um den Stentkörper im zusammengelegten Zustand zu halten, bis der Stent die Implantationsstelle bzw. den Einsetzbereich im Körper erreicht hat, kann dieser in einen Einführkatheter eingeschoben sein. Ist der Stent an die gewünschte Implantationsstelle gelangt, wird der Einführkatheter zurückgezogen, was dazu führt, daß der Stützdraht in die Spiralform einfedert. Durch das Selbstaufwickeln des Stützdrahts wird eine Expansion des zusammengelegten Stentkörpers bis auf das Einsetzvolumen bewirkt, wodurch der Stent sicher an der Implantationsstelle in der Körperhöhle gehalten wird. Besondere Aufspannmittel sind beim erfindungsgemäßen Stent nicht erforderlich.

Im zusammengefalteten, komprimierten bzw. Einführzustand des Stents ist zumindest das distale Ende des Stützdrahts am distalen Einführende des Stents aus dem Stentkörper herausgeführt. Dabei ist der Stützdraht entgegen seiner Federkraft gestreckt. Der langgestreckte Stützdraht kann vor dem Einfedern eine Länge aufweisen, die das Mehrfache der Länge des Stützdrahts nach dem Einfedern betragen kann, insbesondere das wenigstens 1,5-fache. Dabei ist der Stützdraht im Einfuhrzustand am distalen Einführende des Stents zumindest um eine solche Länge aus dem Stentkörper herausgeführt, die das Einfedern in die Spiralform und die Ausbildung eines Stützkörpers ausreichender Länge im Stentkörper ermöglicht. Letztlich verbleibt im komprimierten Zustand im Stent lediglich ein Stützdrahtabschnitt einer solchen Länge, die gleich oder geringfügig größer ist als die Länge des Stentkörpers. Der aus dem Stentkörper herausgeführte Bereich des Stützdrahtes, der die überwiegende Länge des Stützdrahtes ausmacht, muß allerdings nicht notwendigerweise gespannt sein. Letztlich muß nur sichergestellt sein, daß sich der Stützdraht im komprimierten Zustand des Stents nicht in diesen hineinzieht und den Stent damit möglicherweise zumindest teilweise aufweitet. Im Aufspannzustand erstreckt sich der durch Einfedern des Stützdrahtes gebildete Stützkörper jedenfalls vorzugsweise über die gesamte Länge des Stentkörpers, wodurch eine ausreichende Stabilität des Stents über seine gesamte Länge sichergestellt ist.

Der Stützdraht kann aus einer sogenannten Formgedächtnis-Legierung hergestellt sein, vorzugsweise aus einer Nickel-Titan-Legierung, insbesondere aus Nitinol. Das Selbst-Expandieren des Stentkörpers wird dabei durch die Federkraft des Stützdrahtes bewirkt, wobei der Stützdraht aus einer zumindest im wesentlichen gestreckten Form im Bereich des Stentkörpers in eine Spiralform einfedert. Grundsätzlich kann der Stützdraht selbstverständlich auch in andere Formen einfedern, die zur Ausbildung eines Stützgerüstes ausreichender Steifigkeit fuhren. Im Ausführungsfall eines AAA-Stents besteht der Stentkörper besteht aus einem biokompatiblen Material, beispielsweise aus einer PTFE-Membran, insbesondere einer GORE-TEX®-Membran. Hier kann vorgesehen sein, daß der Stützdraht auch innerhalb der Membran, d.h. zwischen zwei Membranschichten, geführt ist. Vorzugsweise liegt der Stützdraht jedoch von Innen gegen die Membran an.

Das proximale Ende des Stützdrahtes ist im Bereich des Einführanfangs mit dem Stentkörper fest verbunden. Dadurch wird das Einfedern des Stützdrahtes bei der Überführung des Stents vom Einführzustand in den Aufspannzustand ernnöglicht, wobei sich der Stützdraht in Richtung zum Einführanfang aufwickelt. Zur Befestigung des Stentkörper mit dem Stützdraht weist der Stentkörper am Einführanfang wenigstens einen innenliegenden Befestigungsring für den Stützdraht auf. Grundsätzlich ist es auch möglich, daß der Stützdraht mit dem Stentkörper verklebt ist, wobei auch andere aus dem Stand der Technik an sich bekannte formschlüssige oder stoffschlüssige Verbindungen zur Befestigung des Stützdrahtes mit dem Stentkörper vorgesehen sein können. Im Aufspannzustand des Stents ist das distale Ende des Stützdrahtes innerhalb des Stentkörpers angeordnet und vorzugsweise unbefestigt. Bevorzugt ist der Stützdraht dabei lediglich am Einführanfang mit dem Stentkörper fest verbunden. Dadurch wird die Handhabung des Stents vereinfacht, wobei es nicht notwendig ist, nach dem Entfernen des Einfühtkatheters und dem Expandieren des Stentkörpers den Stützdraht an seinem distalen Ende zu fixieren. Der Stützdraht wird vorzugsweise allein aufgrund seiner Federkraft gegen die Innenfläche des Stentkörpers gedrückt und dadurch fixiert.

Für eine vereinfachte Führung des Stützdrahtes im Inneren des Stentkörpers können auf der Innenwand des Stentkörpers eine Mehrzahl von Führungsabschnitten vorgesehen sein. Bei den Führungsabschnitten handelt es sich um Schlaufen, in denen der Stützdraht in axialer Richtung frei beweglich geführt ist. Wesentlich ist in diesem Zusammenhang, daß es durch die Führungsabschnitte nicht zu einer Behinderung des Einfederns des Stützdrahtes in die Spiralform kommt, was eine nur unvollständige Expansion des zusammengelegten Stentkörpers zur Folge hätte.

Bei der Erfindung ist wenigstens eine Verstrebung vorgesehen. Die Verstrebung sollte sich vorzugsweise im Inneren des Stentkörpers, d.h. zwischen wenigstens zwei Schichten des Stentkörpers, in Längsrichtung des Stentkörpers erstrecken. Grundsätzlich ist es natürlich auch möglich, daß die Verstrebung von Innen gegen den Stentkörper anliegt und mit diesem zumindest bereichsweise verbunden ist. Die Verstrebung kann beispielsweise durch einen geradlinigen weiteren Stützdraht gebildet werden, der zu einer Stabilisierung des Stentkörpers im Aufspannzustand beiträgt. Als Verstrebung ist ein weiterer Federdraht vorgesehen, der im zusammengelegten Zustand des Stentkörpers in eine langgestreckte Linienform gezogen ist und der sich beim Einfedern zumindest abschnittsweise zusammenzieht und zu einer Versteifung des Stentkörpers im Aufspannzustand des Stents beiträgt.

Damit der expandierte Stent der Neigung des Gefäßes, sich zu verengen, in jedem Fall widersteht oder um ein Einstülpen in eine Aussackung (Aneurysma) zu verhindern, weist der aus dem Stützdraht durch Einfedern gebildete spiralförmige Stützkörper im Aufspannzustand vorzugsweise einen Windungsabstand zwischen 0,2 cm bis 2 cm auf, insbesondere zwischen 0,5 cm bis 1 cm.

Der Stentkörper kann im Einführzustand eine Länge von 5 cm bis 30 cm, vorzugsweise von 8 cm bis 15 cm, aufweisen. In diesem Zusammenhang läßt es die Erfindung bedarfsweise zu, daß der Stent im Einführzustand eine Länge aufweist, die die Verwendung des Stents zur Überbrückung von Gefäßabschnitten unterschiedlicher Länge ermöglicht. Vor oder während einer Operation und in Abhängigkeit von der erforderlichen Länge des Stents kann der Stentkörper bedarfsweise gekürzt werden. Dementsprechend ist auch die Länge des Stützdrahtes entsprechend zu kürzen, wobei der ungekürzte Stützdraht eine ausreichende Länge aufweisen sollte, die eine Verwendung des Stents zur Überbrückung von Gefäßabschnitten unterschiedlicher Länge ermöglicht. Im langgestreckten Zustand des Stützdrahtes kann der Windungsdurchmesser vorzugsweise weniger als 0,5 cm, insbesondere weniger als 0,2 cm, betragen.

Der Stützdraht kann in Form einer archimedischen oder in Form einer logarithmischen Spirale einfedernd ausgebildet sein. Dementsprechend weist der Stentkörper im Aufspannzustand eine Zylinderform oder eine Konusform auf, was den Einsatz in unterschiedlichen Gefäßen des Körpers ermöglicht.

Vorzugsweise ist der Stützdraht auch im Aufspannzustand in der Spiralform noch derart radial vorgespannt, daß auch nach dem Einfedern im Stentkörper weiterhin Federkräfte im Stützdraht in radialer Richtung wirken, die dazu führen, daß sich der Stützdraht eng an den Stentkörper anlegt, diesen über die gesamte Länge stabilisiert und sich im übrigen eine sichere Positionierung des Stents im Blutgefäß ergibt. Dabei nimmt der Stützkörper im Inneren des Stentkörpers nicht seinen maximale aufgrund der Vorspannung des Stützdrahtes möglichen Durchmesser an. Das Volumen des Stützkörpers wird durch das maximale Einsetzvolumen des Stentkörpers auf einen vergleichsweise geringeren Durchmesser begrenzt. Dadurch wird sichergestellt, daß der Stützdraht zu jeder Zeit an der Innenfläche des Stentkörpers anliegt und eine optimale Stützwirkung durch den Stützkörper sichergestellt ist.

Der Stentkörper kann im Aufspannzustand am Einführanfang und/oder am Einführende einen Außenkonus aufweisen. Hier ist von Vorteil, wenn der

Stent an seinem proximalen Ende einen größeren Durchmesser als im übrigen Bereich aufweist, um eine ausreichende Dichtheit bzw. eine sichere Anlage des Stentkörpers gegen die Körperhöhle in diesem Bereich zu gewährleisten. Dies führt dazu, daß der Stent nach der Expansion des Stentkörpers auf das Einsetzvolumen an der Implantationsstelle fixiert wird. In diesem Zusammenhang kann der Stützdraht auch wenigstens zwei Bereiche aufweisen, die in unterschiedlichen Spiralformen einfedern. Durch Einfedern des Stützdrahtes in entsprechende Spiralformen kann eine Expansion des Stentkörpers in einem mittleren Bereich des Stents auf eine Zylinderform und an den Enden des Stents auf eine Konusform bewirkt werden.

Der Stützdraht kann wenigstens eine Sollbruchstelle aufweisen, die es ermöglicht, den Stützdraht in Abhängigkeit von der im Aufspannzustand benötigten Länge des Stents auch in der Körperhöhle zu kürzen. Die erforderliche Länge des Stützdrahtes wird durch die Länge des Stentkörpers im Aufspannzustand vorgegeben, wobei nach dem Einfedern des Stützdrahtes der Stützkörper eine Länge aufweisen sollte, die vorzugsweise der Länge des Stentkörpers entspricht. Dabei kann der Stützdraht vor dem Einführen oder auch endoskopisch nach dem Erreichen der Implantationsstelle gekürzt werden.

Um den Stützdraht zum Einfedern in die Spiralform langsam bewegen zu können, so daß ein definiertes Auffedern gewährleistet ist, weist der Stützdraht am distalen Ende vorzugsweise einen Greifabschnitt für eine Greifeinrichtung auf. Bei dem Greifabschnitt handelt es sich vorzugsweise um eine Schlaufe oder einen Knoten.

Der Stentkörper kann auf der Außenseite Vorsprünge und/oder eine Membran mit hoher Porosität und/oder hoher Oberflächenrauhigkeit zumindest der äußeren Oberfläche aufweisen. Dadurch wird das Anwachsen von Zellen auf dem Stentkörper im Aufspannzustand unterstützt und die Lage des Stents in der Körperhöhle weiter fixiert.

Im übrigen besteht ein wesentlicher Vorteil des erfindungsgemäßen Stents darin, daß der Stentkörper im implantierten Zustand des Stents in einfacher Weise an einer Stelle bereichsweise geöffnet werden kann, um einen Stentkörper eines benachbarten weiteren Stents mit kleinerem Einsetzvolumen in die Öffnung einzuschieben und eine Gefäß-Abzweigung im Körper zu überbrücken. Die Herstellung einer derartigen Abzweigung über einen Stent ist deshalb sehr einfach möglich, da der Stützdraht lediglich am proximalen Ende mit dem Stentkörper verbunden, ansonsten jedoch unverbunden ist. Hierdurch kann der Stentkörper ohne weiteres eingeschnitten und in den Einschnitt der weitere Stent eingesetzt und fixiert werden.

Im übrigen kann vorgesehen sein, daß der Stützdraht nicht über seine gesamte Länge in die Spiralform einfedert, sondern nur abschnittsweise, so daß nach dem Einfedern des Stützdrahtes bzw. nach dem Expandieren des Stentkörpers spiralförmige Abschnitte und im wesentlichen gerade Abschnitte des Stützdrahtes vorliegen, die insbesondere abwechselnd nebeneinander in Längsrichtung des Stentkörpers angeordnet sein können. Im Bereich der nach dem Einfedern vorliegenden geraden Abschnitte des Stützdrahtes ist ein erleichterter Zugang zum Stentkörper möglich, beispielsweise, um das Ende oder den Anfang eines weiteren Stentkörpers an den Stentkörper anschließen zu können.

Im einzelnen gibt es eine Vielzahl von Möglichkeiten, den erfindungsgemäßen Stent auszugestalten und weiterzubilden, wobei einerseits auf die abhängigen Patentansprüche und andererseits auf die nachfolgende detaillierte Beschreibung eines bevorzugten Ausführungsbeispiels der Erfindung unter Bezugnahme auf die Zeichnung verwiesen wird. In der Zeichnung zeigt
- Fig. 1: eine schematische Ansicht eines komprimierten Stents,
- Fig. 2: eine Ansicht des Stents aus Fig. 1 im Aufspannzustand,
- Fig. 3: eine schematische Ansicht einer weiteren Ausführungsform eines Stents im Aufspannzustand und
- Fig. 4: eine schematische Ansicht einer erfindungsgemäßen Ausführungsform eines Stents im Aufspannzustand.

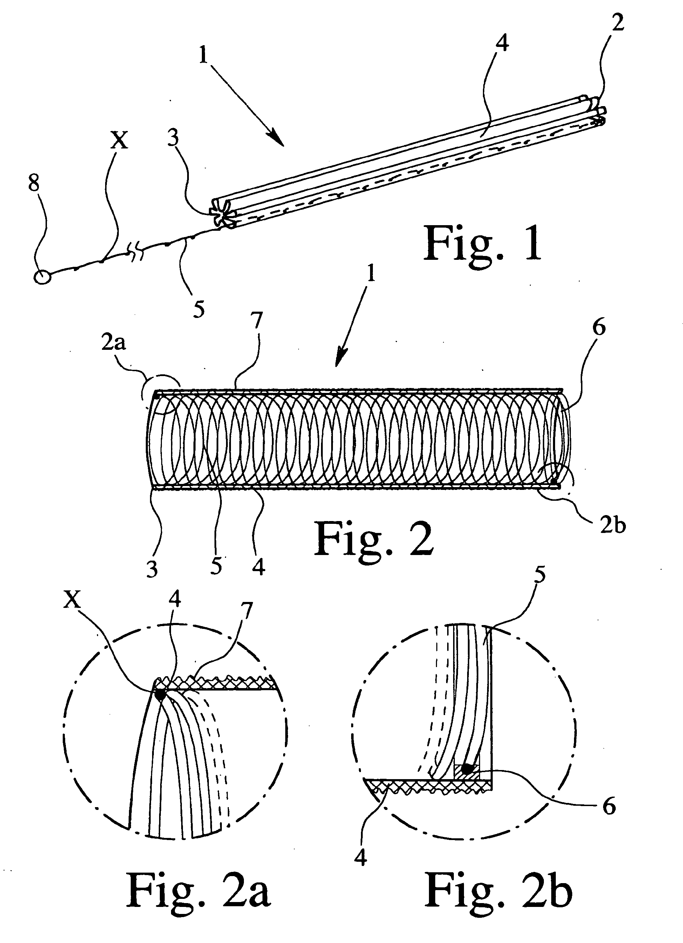

In Fig. 1 ist ein Stent 1 zum Einführen in menschliche Körperhöhlen und insbesondere in Blutgefäße mit einem proximalen Einführanfang 2 und einem distalen Einführende 3 in einem Einführzustand dargestellt. Der Stent 1 weist einen schematisch dargestellten zusammengelegten oder gefalteten Stentkörper 4 und einen im Inneren des Stentkörpers 4 vorgesehenen Stützdraht 5 auf.

Der Stentkörper 4 weist im komprimierten, zusammengefalteten Einführzustand, der in Fig. 1 dargestellt ist, ein vergleichsweise kleines Einführvolumen auf, wobei der Stentkörper 4 in radialer Richtung komprimiert ist. Um den Stent 1 im Einführzustand zu halten, ist ein nicht dargestellter Einführkatheter bzw. eine sogenannte Einführschleuse vorgesehen, in den der Stent 1 zum Einführen in den Körper eingeschoben ist. Der Stent 1 wird in das Gefäß eingeschoben und vorzugsweise unter radiologischer Kontrolle (Bildverstärker) an der gewünschten Stelle positioniert. Zur Überführung des Stents 1 in einen Aufspannzustand wird der Einhhrkatheter zurückgezogen, während der Stent 1 an der Implantationsstelle verbleibt, was aufgrund der Vorspannung des Stützdrahtes 5 zu einem Einfedern des Stützdrahtes 5 und zu einer Expansion bzw. Entfaltung des Stentkörpers 4 führt. Dabei kann das Aufspannen des Stentkörpers 4 durch den Stützdraht 5 definiert vorgenommen werden, wobei das distale Ende über ein entsprechendes Werkzeug langsam in Richtung des Stentkörpers bewegt wird. Grundsätzlich läßt es die Erfindung bedarfsweise zu, daß eine endgültige Verankerung des Stents durch Ballon-Dilatation erfolgen kann.

Wird der Stent 1 innerhalb des Einführkatheters in den Körper eingeschoben, so verhindert der Einführkameter das Aufspannen des Stentkörpers 4. Es ist jedoch grundsätzlich auch möglich, daß der Stentkörper 4 auf einen Katheter aufgeschoben ist, wobei das Aufspannen des Stentkörpers 4 während des Einführens in den Körper durch eine äußere Schutzhülle oder dergleichen verhindert wird. Zur Überführung des Stents 1 in einen Aufspannzustand wird dann zunächst die Schutzhülle zurückgezogen, während der Stent 1 an der Implantationsstelle verbleibt, so daß es zur Entfaltung des Stentkörpers 4 kommt. Nach der Entfaltung des Stentkörpers 4 kann dann der Katheter ebenfalls zurückgezogen werden. Hierzu können an sich aus dem Stand der Technik bekannte Werkzeuge eingesetzt werden.

Darüber hinaus ist es auch möglich, daß der Stützdraht 5 als solcher durch eine Schutzhülle beim Einführen des Stents 1 in den Körper daran gehindert wird, einzufedern, wobei die Schutzhülle zwischen dem äußeren Stentkörper 4 und dem inneren Stützdraht 5 angeordnet sein kann. Zusätzlich kann eine weitere äußere Schutzhülle für den Stentkörper 4 vorgesehen sein, um den Stentkörper 4 beim Einführen des Stents 1 in den Körper in einem gefalteten Zustand zu halten.

In dem in Fig. 2 dargestellten Aufspannzustand weist der Stent 1 ein vergrößertes Einsetzvolumen auf, wobei der Stützdraht 5 an der Innenfläche des Stentkörpers 4 spiralförmig anliegt.

Durch Strecken des Stützdrahtes 5 in Längsrichtung entgegen der Federkraft und durch die damit bewirkte Durchmesserreduzierung des spiralförmigen Stützkörpers ist es im Einführzustand möglich, den Stentkörper 4 auf einen sehr kleinen Einführdurchmesser zusammenzulegen bzw. zu komprimieren, was den Einfübrvorgang wesentlich vereinfacht.

Bei der dargestellten Ausführungsform der Erfindung ist vorgesehen, daß der Stützdraht 5 mit seinem proximalen Ende im Bereich des proximalen Einführendes 2 des Stents 1 mit dem Stentkörper 4 befestigt ist und mit seinem distalen Ende im Einführzustand aus dem Stentkörper 4 am distalen Einführende 3 des Stents 1 herausgeführt ist. Dies ist in Fig. 1 dargestellt.

Im Aufspannzustand ist es vorzugsweise vorgesehen, daß der Stützdraht 5 innerhalb des Stentkörpers 4 angeordnet und unbefestigt ist. Dazu ist es vorzugsweise vorgesehen, den Stützdraht 5 vor dem Überführen des Stents 1 in den Aufspannzustand an der Stelle X zu trennen, wodurch sichergestellt ist, daß das distale Ende des Stützdrahtes 5 nach dem Einfedern innerhalb des expandierten Stentkörpers 4 angeordnet ist. Dies ist in Fig. 2a dargestellt.

Der Stützdraht 5 ist mit seinem proximalen Ende im Bereich des proximalen Einführanfangs 2 des Stents 1 mit dem Stentkörper 4 über einen Befestigungsring 6 fest verbunden. Dies ist in Fig. 2b dargestellt. Zusätzlich kann vorgesehen sein, daß im Bereich des proximalen Einführanfangs 2 des Stents 1 der aus dem Stützdraht 5 durch Einfedern gebildete spiralförmige Stützkörper im Aufspannzustand einen geringeren Windungsabstand aufweist als im mittleren Bereich oder im Bereich des distalen Einführendes 3 des Stents 1 und/oder daß der Stützdraht 5 zickzack- oder wellenförmig an seinem proximalen Ende in die Spiralform einfedert. Dadurch kann ein höherer Anpreßdruck am proximalen Einführanfang 2 des Stents 1 sichergestellt werden.

Der Stützdraht 5 ist als formhaltender Federdraht aus einer Formgedächtnis-Legierung ausgebildet und im Einführzustand zumindest im Bereich des Stentkörpers 4 vorgespannt und zumindest im wesentlichen langgestreckt. Vorzugsweise ist in diesem Zusammenhang ein lösbarer Anschlag zur Fixierung des Stützdrahtes 5 im langgestreckten und aus dem Stentkörper 4 herausgeführten Zustand vorgesehen, der den Stützdraht in der langgestreckten Stellung hält.

Aus einem Vergleich der Fig. 1 und 2 wird deutlich, daß der Stützdraht 5 im Einführzustand im wesentlichen gerade entgegen der Federkraft gestreckt ist und allenfalls Windungen mit großem Windungsabstand aufweist. Im Aufspannzustand weist der aus dem Stützdraht 5 gebildete Stützkörper im Inneren des Stentkörpers 4 eine Länge auf, die im wesentlichen der Länge des Stentkörpers 4 entspricht.

In Fig. 2 ist weiter dargestellt, daß der Stentkörper 4 auf der Außenseite Vorsprünge 7 und/oder eine Membran mit einer hohen Porosität und/oder hohen Oberflächenrauhigkeit zumindest der äußeren Oberfläche aufweist. Dadurch wird das Anwachsen von Zellen nach dem Einsetzen des Stents 1 in eine Körperhöhle unterstützt, was dazu führt, daß der Stent 1 in seiner Lage im Körper fixiert wird.

Darüber hinaus ist Fig. 1 zu entnehmen, daß der Stützdraht 5 an seinem distalen Ende im Einführzustand einen Greifabschnitt 8 aufweisen kann. Der Greifabschnitt 8 ermöglicht es, den Stützdraht 5 beim Einführen und beim Einkürzen mit einem entsprechenden Werkzeug zu halten.

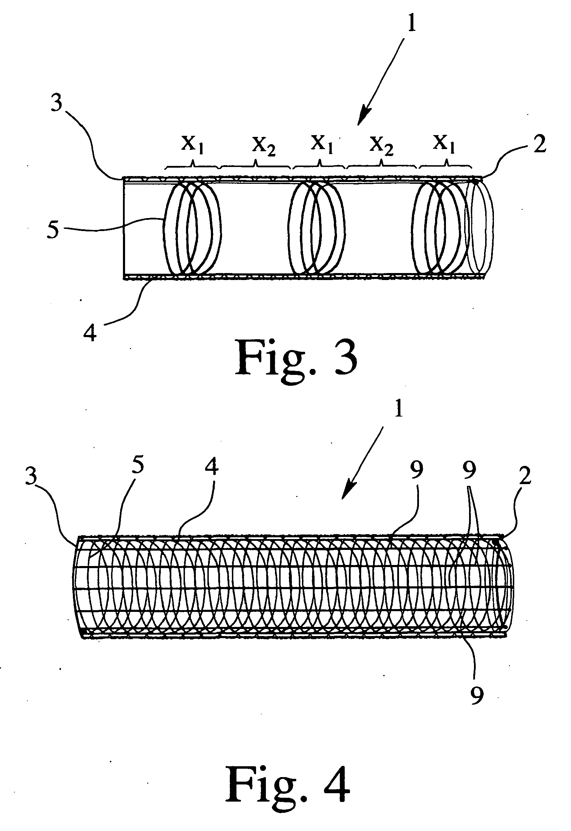

In den Fig. 3 und 4 sind weitere mögliche Ausführungsformen für einen Stent 1 dargestellt, wobei gemäß Fig. 3 der Stützdraht 5 nicht über die gesamte Länge des Stentkörpers 4 in eine Spiralform eingefedert ist. Im Aufspannzustand des Stentkörpers 4 weist der Stützdraht 5 daher zum einen Abschnitte X₁ auf, über deren Länge der Stützdraht 5 in eine Spiralform eingefedert ist. Über die Länge von weiteren Abschnitten X₂ weist der Stützdraht 5 zum anderen eine im wesentlichen langgestreckte Linienform auf. Die Abschnitte X₁ und die weiteren Abschnitte X₂ sind abwechselnd nebeneinander angeordnet Die langgestreckte Form des Stützdrahts 5 im Bereich der weiteren Abschnitte X₂ vereinfacht die Öffnung des Stentkörpers 4 in diesem Bereich und den Anschluß eines weiteren nicht dargestellten Stents an den Stentkörper 4, wobei der Stentkörper des weiteren Stents mit dem Stentkörper 4 verbunden wird.

Gemäß Fig. 4 sind Verstrebungen 9 vorgesehen, die eine gerade Linienform aufweisen und sich in Längsrichtung des Stents 1 erstrecken. Die Verstrebungen 9 sind vorgesehen, um die Steifigkeit des Stents 1 im Aufspannzustand des Stentkörpers 4 zu erhöhen. Die Verstrebungen 9 können zumindest abschnittsweise mit dem Stentkörper 4 verbunden sein oder auch im Inneren des Stentkörpers 4 verlaufen. In Abhängigkeit von der gewählten Applikationsstelle des Stents 1 im Körper können die Verstrebungen 9 mehr oder weniger biegbar ausgebildet sein. Die Verstrebungen 9 sind vorzugsweise als nichteinfedemde Drähte ausgebildet. Es ist jedoch grundsätzlich auch möglich, daß es sich bei den Verstrebungen 9 um Federdrähte handelt, die sich im Aufspannzustand beispielsweise zickzackförmig im Stentkörper 4 erstrecken und im zusammengelegten Zustand in eine langgestreckte gerade Linienform gezogen und aus dem distalen Einführende 3 des Stentkörpers 4 herausgeführt sind.

## Patentansprüche

1. Stent (1) zum Einführen in menschliche Körperhöhlen, insbesondere in Blutgefäße, mit einem proximalen Einführanfang (2) und einem distalen Einführende (3), mit einem hülsenförmigen Stentkörper (4) aus biokompatiblem Material und mit wenigstens einem innerhalb des Stentkörpers (4) vorgesehenen Stützdraht (5) als Stützkörper, wobei der Stützdraht (5) im Aufspannzustand des Stents innenseitig spiralförmig am Stentkörper (4) anliegt, wobei der Stentkörper (4) in einem komprimierten Zustand des Stents zusammengelegt ist und in einen Aufspannzustand expandierbar ist, wobei der Stützdraht (5) mit seinem proximalen Ende im Bereich des proximalen Einführanfangs (2) mit dem Stentkörper (4) fest verbunden ist und mit seinem distalen Ende im komprimierten Zustand aus dem distalen Einführende (3) des Stentkörpers (4) herausgeführt ist, wobei der Stützdraht (5) als formhaltender Federdraht ausgebildet ist, der im komprimierten Zustand zumindest im Bereich des Stentkörpers (4) vorgespannt und in eine langgestreckte gerade Linienform gezogen ist und der sich bei Einfedern zumindest abschnittsweise in die Spiralform in Längsrichtung zusammenzieht und radial spiralförmig expandiert, um eine radiale Expansion des zusammengelegten Stentkörpers (4) bis in den Aufspannzustand zu bewirken, wobei ein Stützdrahtabschnitt des Stützdrahtes (5), der sich vom Einführende (3) des Stentkörpers (4) bis zum Einführanfang (2) des Stentkörpers (4) innerhalb des Stentkörpers (4) erstreckt, im komprimierten Zustand des Stents (1) im wesentlichen die gleiche Länge aufweist wie der Stentkörper (4) und wobei der Stützdraht (5) in axialer Richtung frei beweglich im Inneren des Stentkörpers (4) geführt ist, **dadurch gekennzeichnet, dass** der Stützdraht (5) mit einem am Einführanfang (2) des Stentkörpers (4) vorgesehenen innenliegenden Befestigungsring (6) verbunden ist und dass wenigstens eine sich im Inneren des Stentkörpers (4) in Längsrichtung des Stentkörpers (4) erstreckende Verstrebung (9) vorgesehen ist, wobei es sich bei der Verstrebung (9) um einen Federdraht handelt, der sich im Aufspannzustand im Stentkörper (4) erstreckt und im zusammengelegten Zustand in eine langgestreckte gerade Linienform gezogen und aus dem distalen Einführende (3) des Stentkörpers (4) herausgeführt ist.

2. Stent nach Anspruch 1, **dadurch gekennzeichnet, dass** das distale Ende des Stützdrahtes (5) im Aufspannzustand innerhalb des Stentkörpers (4) angeordnet und unbefestigt ist.

3. Stent nach Anspruch 1 oder 2, **dadurch gekennzeichnet, dass** der aus dem Stutzdraht (5) gebildete spiralförmige Stützkörper im Aufspannzustand einen Windungsabstand zwischen 0,2 cm bis 2 cm aufweist.

4. Stent nach einem der vorhergehenden Ansprüche, **dadurch gekennzeichnet, dass** der Stentkörper (4) im komprimierten Zustand eine Länge von 5 cm bis 30 cm aufweist.

5. Stent nach einem der vorhergehenden Ansprüche, **dadurch gekennzeichnet, dass** der Stützdraht (5) in der Form einer archimedischen oder in der Form einer logarithmischen Spirale einfedernd ausgebildet ist und/oder dass der Stützdraht (5) im Aufspannzustand nach Einfedern in die Spiralform radial vorgespannt ist.

6. Stent nach einem der vorhergehenden Ansprüche, **dadurch gekennzeichnet, dass** der Stentkörper (4) im Aufspannzustand am Einführanfang (2) und/oder am Einführende (3) einen Außenkonus aufweist.

7. Stent nach einem der vorhergehenden Ansprüche, **dadurch gekennzeichnet, dass** der Stützdraht (5) wenigstens eine Sollbruchstelle aufweist und/oder dass der Stützdraht (5) am distalen Ende einen Greifabschnitt (8) für eine Greifeinrichtung aufweist.

8. Stent nach einem der vorhergehenden Anspruch, **dadurch gekennzeichnet, dass** der Stützdraht (5) lediglich abschnittsweise in die spiralform einfedert, so dass nach dem Einfedern spiralförmige Abschnitte (X₁) und im wesentlichen gerade Abschnitte (X₂) des Stützdrahtes (5) vorliegen.

9. Stent nach einem der vorhergehenden Ansprüche, **dadurch gekennzeichnet, dass** auf der Innenfläche des Stentkörpers (4) eine Mehrzahl von Führungsabschnitten zur Führung des Stützdrahtes (5) vorgesehen ist, wobei die Führungsabschnitte als Schlaufen ausgebildet sind, in denen der Stützdraht (5) in axialer Richtung frei beweglich geführt ist.

10. Stent nach Anspruch 9, **dadurch gekennzeichnet, dass** der Stützdraht (5) über die gesamte Länge des Stentkörpers (4) in die Spiralform einfedert.

## Claims

1. A stent (1) for introducing into human body cavities, in particular into blood vessels, with a proximal first end to be introduced (2) and a distal final end to be introduced (3), a sleeve-shaped stent body (4) made of biocompatible material, and at least one support wire (5) provided as a support body within the stent body (4), the support wire (5), when the stent is in the tensioned state, resting against the inside of the stent body (4) in the shape of a spiral, wherein the stent body (4), when the stent is in a compressed state, is collapsed and expandable into a tensioned state, wherein the proximal end of the support wire (5) in the region of the proximal first end to be introduced (2) is firmly attached to the stent body (4) and the distal end of the support wire, when the stent is in the compressed state, extends from the distal final end (3) of the stent body (4) to be introduced, wherein the support wire (5) is embodied as a shape-retaining spring wire which in the compressed state is pre-tensioned at least in the region of the stent body (4) and is drawn into an elongated straight linear shape and contracts in the longitudinal direction into the spiral shape at least in sections upon closing of the spring and expands radially in the form of a spiral in order to effect a radial expansion of the contracted stent body (4) until it reaches the tensioned state, wherein a support wire section of the support wire (5), which extends from the final end to be introduced (3) of the stent body (4) to the first end to be introduced (2) of the stent body (4) within the stent body (4), in the compressed state of the stent (1) has substantially the same length as the stent body (4) and wherein the support wire (5) is guided so as to be freely movable in axial direction in the interior of the stent body (4), **characterized in that** the support wire (5) is connected with an internal fastening ring (6) provided at the first end to be introduced (2) of the stent body (4), and that at least one strut (9) is provided, extending in the interior of the stent body (4) in the longitudinal direction of the stent body (4), wherein the strut (9) is a spring wire which extends in the stent body (4) in the tensioned state and in the contracted state is drawn into an elongated straight linear shape and is guided out from the distal final end to be introduced (3) of the stent body (4).

2. The stent according to Claim 1, **characterized in that** the distal end of the support wire (5) in the tensioned state is arranged inside the stent body (4) and is unsecured.

3. The stent according to Claim 1 or 2, **characterized in that** the spiral-shaped support body formed from the support wire (5) in the tensioned state has a coil spacing between 0.2 cm to 2 cm.

4. The stent according to one of the preceding Claims, **characterized in that** the stent body (4) in the compressed state has a length of 5 cm to 30 cm.

5. The stent according to one of the preceding claims, **characterized in that** the support wire (5) is constructed so as to deflect in the form of an Archimedean or in the form of a logarithmic spiral and/or that the support wire (5) is pretensioned radially into the spiral shape in the tensioned state after deflection.

6. The stent according to one of the preceding claims, **characterized in that** the stent body (4) in the tensioned state has an outer taper at the first end to be introduced (2) and/or at the final end to be introduced (3).

7. The stent according to one of the preceding claims, **characterized in that** the support wire (5) has at least one predetermined breaking point and/or that the support wire (5) has at the distal end a gripping section (8) for a gripper device.

8. The stent according to one of the preceding claims, **characterized in that** the support wire (5) only deflects in sections into the spiral shape, so that after the deflection spiral-shaped sections (X₁) and substantially straight sections (X₂) of the support wire (5) are present.

9. The stent according to one of the preceding claims, **characterized in that** on the inner surface of the stent body (4) a plurality of guide sections is provided for guiding the support wire (5), wherein the guide sections are constructed as loops in which the support wire (5) is guided so as to be freely movable in axial direction.

10. The stent according to Claim 9, **characterized in that** the support wire (5) deflects into the spiral shape over the entire length of the stent body (4).

## Revendications

1. Stent (1) destiné à être introduit dans des cavités du corps humain, en particulier dans des vaisseaux sanguins, comportant une extrémité initiale d'introduction proximale (2) et une extrémité terminale d'introduction distale (3), un corps de stent en forme de manchon (4) en matériau biocompatible et au moins un fil support (5) servant de corps support prévu à l'intérieur du corps de stent (4), le fil support (5) reposant à l'intérieur en forme de spirale sur le corps de stent (4) à l'état de déploiement du stent, le corps de stent (4) étant rétracté en état comprimé du stent et pouvant être gonflé en état de déploiement, le fil support (5) étant relié fixement au corps de stent (4) par son extrémité proximale au niveau de l'extrémité initiale d'introduction proximale (2) et étant sorti par son extrémité distale en état comprimé hors de l'extrémité d'introduction distale (3) du corps de stent (4), le fil support (5) se présentant sous forme d'un fil à ressort à mémoire de forme qui, en état comprimé, est précontraint du moins au niveau du corps de stent (4) et est étiré en une forme linéaire rectiligne étendue en longueur et qui, en se tassant, se rétracte du moins par endroits en forme de spirale dans le sens longitudinal et se gonfle radialement en forme de spirale afin de provoquer une expansion radiale du corps de stent rétracté (4) jusqu'en état de déploiement, une section de fil support du fil support (5) qui s'étend depuis l'extrémité terminale d'introduction (3) du corps de stent (4) jusqu'à l'extrémité initiale d'introduction (2) du corps de stent (4) s'étendant dans le corps de stent (4), présentant en état comprimé du stent (1) sensiblement la même longueur que le corps de stent (4) et le fil support (5) étant guidé de manière librement mobile à l'intérieur du corps de stent (4), **caractérisé en ce que** le fil support (5) est relié à une bague de fixation (6) intérieure prévue à une extrémité initiale d'introduction (2) du corps de stent (4) et qu'au moins un renfort (9) s'étendant à l'intérieur du corps de stent (4) dans le sens longitudinal du corps de stent (4) est prévu, le renfort (9) étant un fil à ressort qui s'étend en état de déploiement dans le corps de stent (4) et étant, en état rétracté, étiré en une forme linéaire rectiligne s'étendant en longueur et étant sorti par l'extrémité initiale d'introduction (3) du corps de stent (4).

2. Stent selon la revendication 1, **caractérisé en ce que** l'extrémité distale du fil support (5), en état de déploiement, est disposée à l'intérieur du corps de stent (4) et n'est pas fixée.

3. Stent selon la revendication 1 ou 2, **caractérisé en ce que** le corps support en forme de spirale formé à partir du fil support (5), en état de déploiement, présente une distance entre ses spires comprise entre 0,2 cm et 2 cm.

4. Stent selon une des revendications précédentes, **caractérisé en ce que** le corps de stent (4), en état comprimé, présente une longueur de 5 cm à 30 cm.

5. Stent selon une des revendications précédentes, **caractérisé en ce que** le fil support (5) est réalisé de manière à se tasser en forme d'une spirale d'Archimède ou en forme de spirale logarithmique et/ou que le fil support (5), en état de déploiement, est précontraint radialement en forme de spirale après s'être tassé.

6. Stent selon une des revendications précédentes, **caractérisé en ce que** le corps de stent (4), en état déployé, présente un cône extérieur à l'extrémité initiale d'introduction (2) et/ou à l'extrémité terminale d'introduction (3).

7. Stent selon une des revendications précédentes, **caractérisé en ce que** le fil support (5) présente au moins un point de rupture théorique et/ou que le fil support (5) présente à son extrémité distale une section de préhension (8) pour un dispositif de préhension.

8. Stent selon une des revendications précédentes, **caractérisé en ce que** le fil support (5) se tasse simplement par endroits en forme de spirale de manière à obtenir après le tassement des sections en forme des spirales (X₁) et des sections sensiblement rectilignes (X₂) du fil support (5).

9. Stent selon une des revendications précédentes, **caractérisé en ce qu'**il est prévu sur la surface intérieure du corps de stent (4) une pluralité de sections de guidage pour guider le fil support (5), les sections de guidage se présentant sous forme de boucles dans lesquelles le fil support (5) est guidé de manière librement mobile dans le sens axial.

10. Stent selon la revendication 9, **caractérisé en ce que** le fil support (5) se tasse en forme de spirale sur toute la longueur du corps de stent (4).
